# EUROPÄISCHE PATENTSCHRIFT

(11) **EP 1 340 461 B1**
(45) Veröffentlichungstag und Bekanntmachung des Hinweises auf die Patenterteilung: **29.06.2005**
(21) Anmeldenummer: 03003805.3
(22) Anmeldetag: 20.02.2003
(51) Int. Cl.: A61B 17/08, A61B 19/00

(54) **Instrument zur Gewebedehnung der Haut**
Instrument for stretching skin
Instrument pour étirer la peau

(30) Priorität: 01.03.2002 DE 10209122
(43) Veröffentlichungstag der Anmeldung: 03.09.2003
(73) Patentinhaber: Fleischmann, Wilhelm, Dr. med., 74321 Bietigheim-Bissingen (DE)
(72) Erfinder: Fleischmann, Wilhelm, Dr. med., 74321 Bietigheim-Bissingen (DE)
(74) Vertreter: Westphal, Mussgnug & Partner

(56) Entgegenhaltungen:
- SU-A- 1 412 751
- US-A- 5 814 067
- US-B1- 6 254 624

## Beschreibung

Die Erfindung betrifft ein Instrument zur Gewebedehnung der Haut gemäß dem Oberbegriff des Anspruchs 1.

Zum Schließen größerer Wunden und Hautdefekte ist es z. B. aus der US 5,486,196 und der US 5,618,310 bekannt, die die Wunde oder den Hautdefekt umgebende Haut mechanisch zu dehnen, wodurch eine verstärkte Gewebeneubildung bewirkt wird. Für diese Hautdehnung (Skin Stretching) werden Distraktoren verwendet, die mit Zugmitteln in der Haut verankert werden. Über diese Zugmittel wird die Zugkraft zur Dehnung des Hautgewebes in die Haut eingeleitet. Eine hohe Zugkraft bewirkt dabei eine stärkere Gewebeproliferation. Eine hohe Zugkraft bedeutet dabei jedoch auch, dass auf der Druckseite vor den Zugmitteln das Hautgewebe komprimiert wird. Bei dem aus der US 5,486,196 bekannten Instrument wird die Zugkraft auf das Hautgewebe über eine lange Interdermalnadel übertragen, die parallel zu dem Wundrand in der Haut verankert ist. Dadurch können höhere Zugkräfte auf das Hautgewebe übertragen werden, ohne dass der Druck auf der Druckseite der langen Nadel über den kritischen Verschlussdruck des Gewebesystems ansteigt, der zu einer Ischämie des Gewebes führt. Bei dem Instrument der US 5,618,310 wird die Zugkraft mittels einer punktuell in die Haut eingestochenen Nadel in das Hautgewebe eingeleitet. Es wird daher die Zugkraft so begrenzt, dass auf der Druckseite der Nadel der zu Ischämie führende kritische Verschlussdruck nicht erreicht wird. Diese Begrenzung der Zugkraft bedeutet auch eine Begrenzung der Hautdehnung, so dass eine optimale Gewebeneubildung nicht erreicht wird.

Ein Instrument der eingangs genannten Gattung ist aus der US-A-5,814,067 bekannt. Dieses Instrument weist zwei gegeneinander verstellbare Backen auf, die Hakenaufnehmer aufweisen, in welche Hakenmodule eingesetzt werden. Die Hakenmodule tragen jeweils wenigstens einen Haken, der zur Verankerung des Instruments in die Haut eingestochen wird. Die Haken sind in einer quer zur Richtung der auf die Haut ausgeübten Zugkraft in den Hakenaufnehmern der Backen angeordnet. Sämtliche Haken üben stets jeweils gleichzeitig die gleiche Zugkraft auf die Haut aus. Diese Zugkraft kann auf einen hohen Wert eingestellt werden, um eine große Gewebeproliferation zu bewirken. Da hierbei jedoch ein punktueller Druck auf die Haut ausgeübt wird, müssen Zeitintervalle eingelegt werden, in denen keine Zugkraft ausgeübt wird.

Der Erfindung liegt die Aufgabe zugrunde, ein Instrument zur Verfügung zu stellen, mit welchem zur Gewebedehnung der Haut eine hohe Zugkraft punktuell in die Haut eingeleitet werden kann, ohne dass die Zugmittel eine Schädigung des Gewebes auf der Druckseite verursachen.

Diese Aufgabe wird erfindungsgemäß gelöst durch ein Instrument mit den Merkmalen des Anspruches 1.

Vorteilhafte Ausführungen der Erfindung sind in den Unteransprüchen angegeben.

Der wesentliche Gedanke der Erfindung besteht darin, über die punktuell in die Haut verankerten Haken eine zyklisch wechselnde Zugkraft in die Haut einzuleiten. Dabei wechselt die Zugkraft vorzugsweise zwischen zwei unterschiedlichen Werten. Der erste Wert der Zugkraft wird möglichst groß gewählt, um eine möglichst hohe Gewebeneubildung zu stimulieren. Dieser erste Wert der Zugkraft wird nach oben im Wesentlichen nur durch die Reißfestigkeit der Haut begrenzt. Der erste Wert wird dabei bewusst so hoch gewählt, dass auf der Druckseite der Zugmittel der kritische Verschlussdruck des venösen Kapillarsystems der Haut überschritten wird und Ischämie bewirkt wird. Diese hohe Zugkraft wird über eine Zeitperiode aufrecht erhalten, die unter der Ischämietoleranzdauer des Hautgewebes liegt. Eine länger als diese Toleranzdauer anhaltende Ischämie führt zu einem druckbedingten Absterben des Hautgewebes und damit zu einer Nekrose auf der Druckseite der Haken. Die Ischämietoleranzdauer des Hautgewebes liegt bei etwa 6 bis 7 Stunden. Bevor diese Ischämietoleranzdauer erreicht und eine Nekrose des Hautgewebes auf der Druckseite der Haken zu befürchten ist, wird die Zugkraft auf den zweiten Wert abgesenkt, der so gewählt wird, dass der auf der Druckseite von den Haken auf das Gewebe ausgeübte Druck unter dem kritischen Verschlussdruck von etwa 20-40 mm Hg liegt. Durch diese Druckreduzierung wird die Blutperfusion in das Gewebe vor dem Haken wieder freigegeben und eine schnelle maximale Reperfusion des Gewebes tritt ein. Diese Reperfusion erfolgt bereits nach etwa 15 Sekunden, so dass nach etwa 0,5 bis 5 Minuten eine vollständige Perfusion und Durchblutung des Gewebes auf der Druckseite der Haken sicher gestellt ist und die Zugkraft wieder auf den hohen ersten Wert gebracht werden kann. Durch diese zyklische Änderung der Zugkraft kann über eine lange Zeitdauer von bis zu einigen Tagen eine sehr hohe Zugkraft für die Gewebedehnung der Haut eingesetzt werden, ohne dass eine Schädigung der Haut und insbesondere eine Nekrose des Gewebes auf der Druckseite der Haken eintritt. Das Instrument eignet sich daher insbesondere für die Langzeit-Hautdistraktion zum Verschließen großflächiger Hautdefekte.

Gemäß der Erfindung werden zwei Gruppen von Haken in der Haut verankert, wobei die Haken der beiden Gruppen nebeneinander in einer Reihe quer zur Richtung der Zugkraft und vorzugsweise alternierend angeordnet sind. Über die eine Gruppe der Haken wird die erste hohe Zugkraft in die Haut eingeleitet, während die zweite Gruppe von Haken entlastet wird und nur die zweite niedrige Zugkraft einleitet. Die beiden Gruppen von Haken wechseln dabei zeitlich periodisch in dem Wert der Zugkraft ab, wobei die Periodendauer des Wechsels kleiner ist als die Ischämietoleranzdauer des Hautgewebes. Es wird dadurch ständig durch eine der Gruppen der Haken die maximale Zugkraft auf das Gewebe ausgeübt, so dass die für die Gewebeneubildung optimale Zugkraft ohne Unterbrechung einwirkt. Andererseits sind die Haken jeweils abwechselnd zugentlastet, so dass sich das Gewebe auf der Druckseite der Haken jeweils während dieser Entlastungsphase vollständig regenerieren kann.

Die Haken, die in die Haut eingestochen werden, sind als Hakenmodule in einem Hakenaufnehmer angeordnet, wobei der Hakenaufnehmer wenigstens zwei Hakenmodule aufnimmt, so dass jeweils zumindest ein Haken für jede der beiden Gruppen vorhanden ist. Die Hakenmodule werden in dem Hakenaufnehmer vorzugsweise alternierend durch geeignete Stellmittel in Richtung der Zugkraft vorgeschoben. Auf die Hakenaufnehmer wird konstant die hohe Zugkraft mit dem ersten Wert ausgeübt. Diese Zugkraft wird über die Haken der jeweils vorgeschobenen Hakenmodule in die Haut eingeleitet. Die Haken der jeweils nicht vorgeschobenen Hakenmodule sind auf diese Weise entlastet. Die entlasteten Hakenmodule werden durch die Elastizität der Haut zurückgeschoben, bis sie keine wesentliche Zugkraft mehr auf die Haut ausüben. Alternativ können die entlasteten Hakenmodule durch eine Federkraft zurückgezogen werden. Ebenso ist es möglich, auch das Zurückfahren der entlasteten Hakenmodule zwangsgesteuert durchzuführen.

Die Verstellung der Hakenmodule, d. h. das Vorschieben der die Zugkraft übertragenden Hakenmodule und das Freigeben bzw. das Zurückfahren der entlasteten Hakenmodule kann in unterschiedlicher Weise erfolgen. Da es im Hinblick auf die Ischämietoleranzdauer von etwa 6-7 Stunden ausreichend ist, den Wechsel zwischen den zwei Gruppen von Zugmitteln im Abstand von einigen Stunden durchzuführen, ist eine einfache manuelle Verstellung praktikabel. Für eine automatische Langzeitbehandlung ist auch ein Antrieb der Verstellung über einen Elektromotor oder eine Pneumatik möglich, die eine automatische Steuerung zulassen.

Im Folgenden wird die Erfindung anhand von in der Zeichnung dargestellten Ausführungsbeispielen näher erläutert. Es zeigen
- Fig. 1: eine Seitenansicht eines Instruments gemäß der Erfindung,
- Fig. 2: einen Schnitt längs der Linie A-A in Fig. 1,
- Fig. 3: eine Fig. 2 entsprechende Ansicht eines Hakenaufnehmers in einer zweiten Ausführung,
- Fig. 4: einen Schnitt längs der Linie B-B in Fig. 3 und
- Fig. 5: eine den Fig. 2 und 3 entsprechende Darstellung eines Hakenaufnehmers in einer dritten Ausführung.

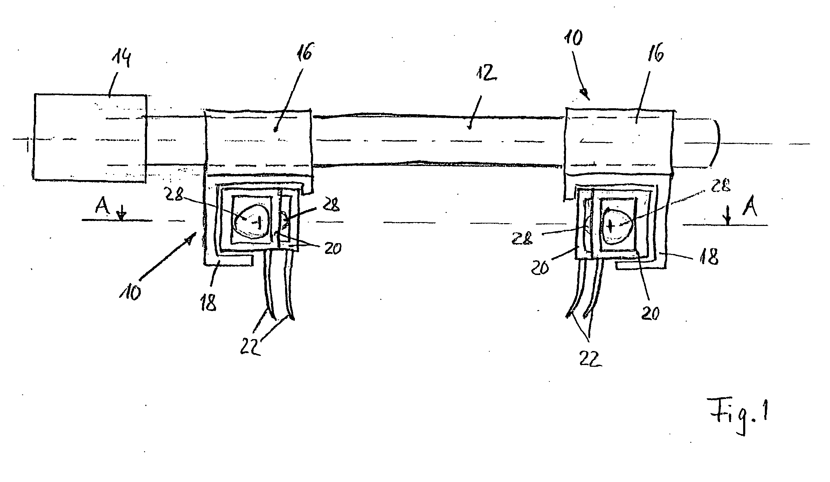

In den Fig. 1 ist ein Instrument zur Gewebedehnung der Haut, ein sog. Haut-Distraktor, dargestellt. Der grundsätzliche Aufbau eines solchen Haut-Distraktors ist an sich bekannt und z. B. in der US 5,486,196 oder der DE 44 44 130 A1 beschrieben. Der Haut-Distraktor weist zwei Backen 10 auf, die mittels geeigneter Stellmittel geführt gegeneinander bewegbar sind. Die Backen 10 und die Stellmittel sind in der Fig. 1 nur schematisch angedeutet. Die Stellmittel können bspw. eine Gewindespindel 12 aufweisen, die mittels eines Antriebs 14 angetrieben wird. Der Antrieb 14 kann ein manuell betätigbarer Rändelknopf oder auch ein gesteuerter motorischer Antrieb sein. Die Backen 10 sitzen in diesem Falle mittels Gewindebuchsen 16 auf Gewindespindel 12. Die Gewindebuchsen 16 weisen gegenläufige Gewinde auf, so daß sich bei einem Drehsinn der Gewindespindel 12 die Backen 10 aufeinander zu bewegen, während sie bei dem entgegengesetzten Drehsinn der Gewindespindel 12 auseinander laufen. Die Backen 10, ihre Führung und ihre Stellmittel können in beliebiger an sich bekannter Weise ausgebildet sein und sind insoweit nicht Gegenstand der Erfindung.

Die Backen 10 weisen jeweils einen Hakenaufnehmer 18 auf, in welchen Hakenmodule 20 eingesetzt werden können. Die Hakenaufnehmer 18 und die Hakenmodule 20 sind so ausgebildet, daß wenigstens zwei Hakenmodule 20 nebeneinander in den Hakenaufnehmer 18 eingesetzt werden können. In dem dargestellten Ausführungsbeispiel sind jeweils fünf Hakenmodule 20 in einen Hakenaufnehmer 18 eingesetzt. Die Hakenaufnehmer 18 sind an den Backen 10 in der Weise angeordnet, daß die in die Hakenaufnehmer 18 eingesetzten Hakenmodule 20 in einer Reihe nebeneinander sitzen, wobei diese Reihe quer zu dem Verstellweg der Bakken 10, d. h. in dem Ausführungsbeispiel der Fig. 1 quer zu der Gewindespindel 12, verläuft. Die Hakenmodule 20 tragen Haken 22. Vorzugsweise weist jedes Hakenmodul 20 nur einen Haken 22 auf, ggf. können die Hakenmodule 20 jedoch auch einige wenige Haken 22 aufweisen. Die Haken 22 werden in die Haut eingestochen und bilden punktuell in der Haut verankerbare Zugmittel. Werden die Backen 10 nach dem Einstechen der Haken in die Haut gegeneinander bewegt, so wird hierdurch über die Haken 22 punktuell die Zugkraft in die Haut eingeleitet, die zur Stimulation der Gewebedehnung erforderlich ist.

In den dargestellten Ausführungsbeispielen sind die Hakenaufnehmer 18 als Hohlprofile ausgebildet, vorzugsweise als Vierkant-Hohlprofile, in welche die Hakenmodule 20 einschiebbar sind. Die Hakenmodule 20 weisen einen auf das Vierkant-Hohlprofil der Hakenaufnehmer 18 abgestimmten Querschnitt auf, so daß sie in später beschriebener Weise in den Hakenaufnehmern 18 geführt senkrecht zu der Profillängsachse beweglich sind, d. h. in der Richtung der Zugkraft bzw. in der Verstellrichtung der Backen 10.

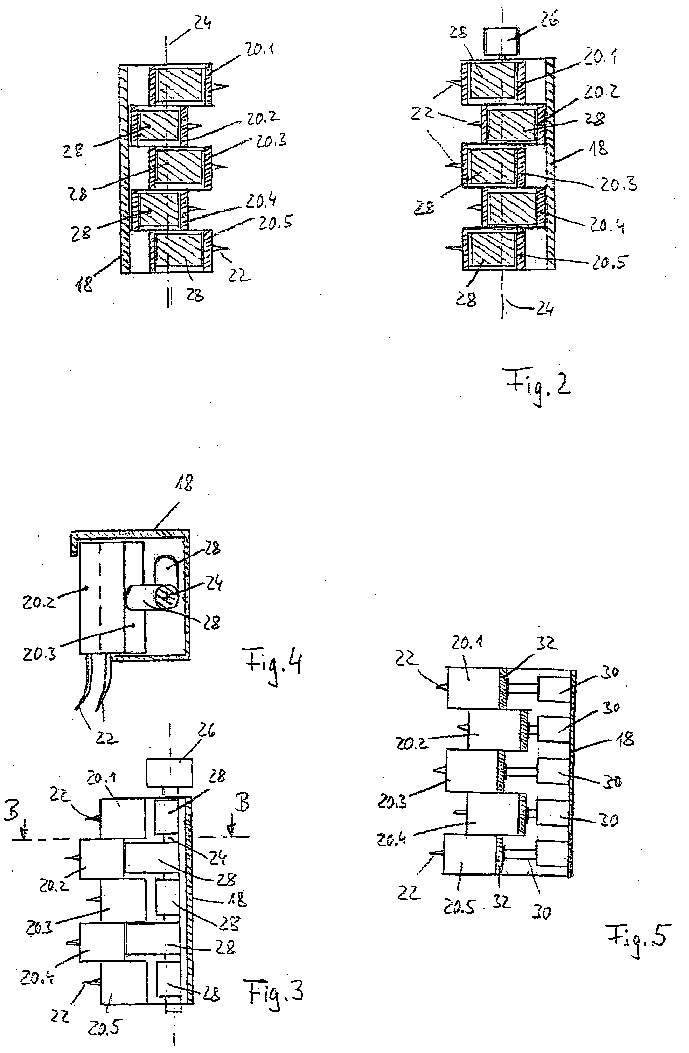

Wie insbesondere aus den Fig. 2, 3 und 5 ersichtlich ist, sind die Hakenmodule 20 in den Hakenaufnehmern 18 in zwei Gruppen unterteilt, wobei sich die Hakenmodule 20 der beiden Gruppen in den Hakenaufnehmern 18 alternierend abwechseln. Die Hakenmodule 20 der einen Gruppe, in den Ausführungsbeispielen die Hakenmodule 20.1, 20.3 und 20.5, sind somit gegenüber den Hakenmodulen 20 der zweiten Gruppe, in der Zeichnung den Hakenmodulen 20.2 und 20.4 auf Lücke versetzt angeordnet. Die Hakenmodule 20.1, 20.3 und 20.5 und die Hakenmodule 20.2 und 20.4 der zweiten Gruppe sind jeweils gruppenweise gemeinsam gegeneinander bewegbar, wie dies in den Fig. 2, 3 und 5 dargestellt ist.

Diese gegenseitige Verschiebung der Hakenmodule 20 mit den Haken 22 ermöglicht das folgende Verfahren für die Gewebedehnung der Haut.

Die Haken 22 werden in die zu dehnende Haut eingestochen. Bspw. werden die Haken 22 der beiden Backen 10 an den einander gegenüber liegenden Rändern eines großflächigen Hautdefektes oder einer Wunde in die Haut eingestochen. Die Backen 10 werden dann mittels der Stellmittel 12, 14 gegeneinander bewegt, um die Wundränder einander anzunähern und die Haut außerhalb der Wundränder zu dehnen. Erfindungsgemäß erfolgt dabei der Antrieb der Backen 10 in der Weise, daß über die Haken 22 eine sehr hohe Zugkraft in die Haut eingeleitet wird. Die Zugkraft darf lediglich die Reißfestigkeit der Haut, die bei ca. 15 N/mm² liegt, nicht überschreiten, da anderenfalls die Haken 22 in der Haut ausreißen würden. Da die Hakenmodule 20 der einen Gruppe in der Zugrichtung vorgeschoben sind, wird diese hohe Zugkraft im Wesentlichen nur über die Haken 22 der vorgeschobenen Hakenmodule in die Haut eingeleitet, d. h. in den Darstellungen der Fig. 2 und 5 durch die Hakenmodule 20.1, 20.3 und 20.5 und in der Darstellung der Fig. 3 durch die Hakenmodule 20.2 und 20.4. Die in den Hakenaufnehmer 18 nebeneinander angeordneten Haken 22 weisen einen gegenseitigen Abstand von etwa 5-15 mm auf. Daher wird die Haut durch die Gruppe der vorgeschobenen Hakenmodule über die ganze Breite des Hakenaufnehmers 18 gespannt und gedehnt, während die zwischen den vorgeschobenen Hakenmodule 20.1, 20.3 und 20.5 liegenden zurückgezogenen Hakenmodule 20.2 und 20.4 über ihre Haken 22 nur eine geringe oder überhaupt keine Zugkraft auf die Haut ausüben.

Die hohe Zugkraft, die über die vorgeschobenen Haken 22 der Hakenmodule 20.1, 20.3 und 20.5 (Fig. 2) in die Haut eingeleitet wird, hat zur Folge, daß auf der Druckseite der Haken 22 auf das Hautgewebe ein Kompressionsdruck ausgeübt wird, der den kritischen Verschlußdruck des venösen Kapillarsystems des Hautgewebes, der bei etwa 20-40 mm/Hg liegt, überschreitet. Diese Zugbelastung des Gewebes wird daher nur für eine Zeitperiode aufrecht erhalten, die deutlich unter der Ischämietoleranzdauer des Hautgewebes von ca. 7 Stunden liegt. Diese Zeitperiode liegt zwischen einigen Minuten und einigen wenigen Stunden. Eine kürzere Zeitperiode von wenigen Minuten, z. B. unter 30 Minuten, ist insbesondere bei einem automatisch gesteuerten Betrieb möglich. Eine längere Zeitperiode von etwa 1-3 Stunden wird wegen des erforderlichen Personaleinsatzes bei einer manuellen Einstellung bevorzugt. Nach dieser Zeitperiode werden die Hakenmodule 20 der beiden Gruppen gegeneinander verschoben, so daß nun die Hakenmodule 20 der ersten Gruppe und der zweiten Gruppe ihre Position vertauschen. Die Hakenmodule 20.1, 20.3 und 20.5 der ersten Gruppe bewegen sich aus der in Fig. 2 dargestellten Position nach hinten und die Hakenmodule 20.2 und 20.4 der zweiten Gruppe werden nach vorn geschoben, so daß die in Fig. 3 dargestellte Position eingenommen wird. Nun wird die hohe Zugbelastung, die durch die Verstellung der Backen 10 bewirkt wird, durch die Haken 22 der zweiten Gruppe in die Haut eingeleitet. Die Haken 22 der zurückgezogenen Hakenmodule 20 sind entlastet und üben auf das auf ihrer Druckseite liegende Hautgewebe keinen oder nur noch einen geringen Kompressionsdruck aus. Dieser Kompressionsdruck liegt deutlich unter dem kritischen Verschlußdruck des venösen Kapillarsystems des Gewebes, so daß die Blutperfusion in das Gewebe auf der Druckseite der Haken 22 freigegeben wird und eine schnelle Wiederdurchblutung dieses Gewebes erfolgt.

Durch den in regelmäßigen Zeitabständen erfolgenden Positionswechsel der Hakenmodule 20.1, 20.3 und 20.5 der ersten Grupe mit den Hakenmodulen 20.2 und 20.4 der zweiten Gruppe ist es möglich, die Hautdehnung über eine lange Behandlungsdauer von Stunden und Tagen durchzuführen, wobei stets über die gesamte Breite der Hakenaufnehmer 18 die maximale Zugbelastung auf das Gewebe ausgeübt wird. Die punktuelle Belastung wechselt dabei jedoch in regelmäßigen Zeitabständen zwischen den Haken 22 der ersten Gruppe und den Haken 22 der zweiten Gruppe, so daß trotz der hohen Zugbelastung eine Gewebeschädigung wegen Mangeldurchblutung nicht eintritt.

Die gegenseitige Verschiebung der Hakenmodule 20 der ersten und der zweiten Gruppe kann in verschiedenen Ausführungen realisiert werden.

In dem Ausführungsbeispiel der Fig. 1 und 2 weisen die Hakenmodule 20 die Form eines rechteckigen Hohlprofils auf. Durch das Hohlprofil der Hakenmodule 20 ist eine Stellwelle 24 hindurchgeführt, die an ihrem einen Ende einen Stellantrieb 26 aufweist. Der Stellantrieb 26 ist in Fig. 2 nur schematisch dargestellt. Es kann sich bei dem Stellantrieb 26 um einen Drehknopf für eine manuelle Verstellung handeln oder um einen gesteuerten motorischen Antrieb für eine automatische Verstellung. Auf der Stellwelle 24 sitzen Stellnocken 28, wobei in jedem Hakenmodul 20 eine zugeordnete Stellnocke 28 vorgesehen ist. Die Stellnocken 28 sind vorzugsweise als sog. Gleichdick ausgebildet und liegen an der vorderen und der hinteren Profilinnenfläche der Hakenmodule 20 an. Die den Hakenmodulen 20.1, 20.3 und 20.5 der ersten Gruppe zugeordneten Stellnocken 28 sind gegenüber den Hakenmodulen 20.2 und 20.4 der zweiten Gruppe zugeordneten Stellnocken 28 um 180° im Winkel versetzt angeordnet, wie aus den Fig. 1 und 2 ersichtlich ist.

Eine Drehung der Stellwelle 24 um 180° führt in dieser Ausführung zu einer Vertauschung der Positionen der Hakenmodule 20.1, 20.3 und 20.5 der ersten Gruppe mit den Hakenmodulen 20.2 und 20.4 der zweiten Gruppe. Da die Stellnocken 28 an der vorderen und der hinteren Innenwandfläche der Hakenmodule 20 anliegen, werden die Hakenmodule 20 sowohl bei der Vorwärtsbewegung als auch bei der Rückwärtsbewegung durch die Stellnokken 28 zwangsgeführt. Die Entlastung der Backen 22 der zurückgezogenen Hakenmodule 20 erfolgt somit zwangsweise und unabhängig von der durch die Haut auf diese Haken ausgeübten elastischen Rückstellkraft.

In den Fig. 3 und 4 ist eine abgewandelte Ausführung dargestellt.

In dieser Ausführung verläuft die Stellwelle 24 hinter den Hakenmodulen 20. Auf der Stellwelle 24 sitzen Stellnocken 28, die jeweils den einzelnen Hakenmodulen 20 zugeordnet sind. Dabei sind die Stellnocken 28, die den Hakenmodulen 20.1, 20.3 und 20.5 der ersten Gruppe zugeordnet sind, gegenüber den Stellnocken 28, die den Hakenmodulen 20.2 und 20.4 der zweiten Gruppe zugeordnet sind, um 90° auf der Stellwelle 24 versetzt angeordnet, wie in Fig. 4 zu erkennen ist. In der Darstellung der Fig. 3 und 4 sind die Stellnocken 28 der Hakenmodule 20.2 und 20.4 in Eingriff und schieben diese Hakenmodule in die vorgeschobene Position. Die Stellnocken 28 der Hakenmodule 20.1, 20.3 und 20.5 sind dagegen zurückgeschwenkt, so daß sie die zugehörigen Hakenmodule freigeben. Bei einer Drehung der Stellwelle 24 um 90° (in Fig. 4 im Gegenuhrzeigersinn) kommen die Stellnocken 28 der anderen Gruppe in Eingriff und schieben die zugehörigen Hakenmodule 20.1, 20.3 und 20.5 in die aktive Position, während die Stellnocken 28 der Hakenmodule 20.2 und 20.4 zurückgeschwenkt werden und die zugehörigen Hakenmodule freigeben.

In dieser Ausführung werden die Hakenmodule 20 durch die Stellnocken 28 nur zwangsweise in die vorgeschobene Position bewegt und in dieser gehalten. Die nicht aktiven Hakenmodule. 20 werden von den zugehörigen Stellnocken 28 nur freigegeben, so daß sie von diesen nicht mehr abgestützt werden. Die Hakenmodule 20 bewegen sich in dieser Ausführung aufgrund der auf ihre Haken 22 einwirkenden elastischen Rückstellkraft der Haut zurück. Die entlasteten Hakenmodule bewegen sich dabei unter der Wirkung der Hautelastizität soweit zurück, bis ihre Haken 22 keine wesentliche Kraft mehr auf die Haut ausüben. Dadurch fällt für die entlasteten Hakenmodule 20 der auf der Druckseite der Haken 22 auf das Hautgewebe ausgeübte Kompressionsdruck praktisch auf Null ab.

In Fig. 5 ist eine weitere Ausführung dargestellt, bei welcher den einzelnen Hakenmodulen 20 jeweils gesonderte Stellmittel 30 zugeordnet sind. Diese Stellmittel können in beliebiger Weise ausgebildet sein, z. B. als Stellschrauben oder wie in Fig. 5 dargestellt als pneumatische Zylinder-Kolben-Stellmittel. Da die Hakenmodule 20 jeweils mit ihren zugeordneten Stellmitteln 30 individuell einstellbar sind, ist in dieser Ausführung eine flexiblere Verteilung der Zugkräfte über die Breite des Hakenaufnehmers 18 möglich. Außerdem eignet sich eine solche Verstellung insbesondere auch für eine Automatisierung. Greifen die Stellmittel 30 zusätzlich über ein elastisches Polster 32 an den Hakenmodulen 20 an, wie dies in Fig 5 angedeutet ist, so können sich die Hakenmodule 20 mit ihren jeweiligen Haken 22 unterschiedlichen Hautspannungen anpassen und diese ausgleichen.

Bei allen Ausführungen der Erfindung wird die die Gewebedehnung bewirkende Zugkraft über die Verstellung der Backen 10 erzeugt. Diese Zugkraft wird insbesondere auch bei einer Langzeitdehnung konstant in das Gewebe eingeleitet. Die Einleitung der Zugkraft in das Gewebe wird jedoch zeitlich periodisch wechselnd auf verschiedene Haken verteilt. Die Haken werden dadurch in regelmäßigen Zeitabständen entlastet, so daß in ihrem Druckbereich die Gewebedurchblutung vollständig wiederhergestellt wird und keine Nekrosen auftreten.

### Bezugszeichenliste

- 10: Backen
- 12: Gewindespindel
- 14: Antrieb
- 16: Gewindebuchsen
- 18: Hakenaufnehmer
- 20: Hakenmodule
- 22: Haken
- 24: Stellwelle
- 26: Stellantrieb
- 28: Stellnocken
- 30: Stellmittel
- 32: Polster

## Patentansprüche

1. Instrument zur Gewebedehnung der Haut, mit Backen (10), mit in Hakenaufnehmer (18) der Backen (10) einsetzbaren Hakenmodulen (20), die jeweils wenigstens einen Haken (22) tragen, wobei die Backen (10) gegeneinander bewegbar sind, um über die in die Haut eingestochenen Haken (22) eine Zugkraft in die Haut einzuleiten, und wobei die Hakenmodule(20) in einer quer zur Richtung der Zugkraft verlaufenden Reihe in dem Hakenaufnehmer (18) angeordnet sind,
und die jeweils in einem Hakenaufnehmer (18) in der quer zur Richtung der Zugkraft verlaufenden Reihe angeordneten Hakenmodule (20.1, 20.3, 20.5 und 20.2, 20.4) in wenigsten zwei Gruppen unterteilt sind, **dadurch gekennzeichnet, dass** die Hakenmodule (20.1, 20.3, 20.5) der einen Gruppe gegen die Hakenmodule (20.2, 20.4) der wenigstens einen anderen Gruppe in Richtung der Zugkraft gegeneinander versetzt in dem Hakenaufnehmer (18) angeordnet sind und zur Vertauschung ihrer gegenseitigen Position in Richtung der Zugkraft gegeneinander bewegbar sind.

2. Instrument nach Anspruch 1,
**dadurch gekennzeichnet, dass** jeweils Hakenmodule (20.1, 20.3, 20.5) der ersten Gruppe und Hakenmodule (20.2, 20.4) der zweiten Gruppe abwechselnd in der quer zur Richtung der Zugkraft verlaufenden Reihe in dem Hakenaufnehmer (18) angeordnet sind.

3. Instrument nach Anspruch 2,
**dadurch gekennzeichnet, dass** die Hakenmodule (20) mit Stellmitteln (26, 28, 30) in ihre in Richtung der Zugkraft vorgeschobene Position zwangsweise verstellbar sind.

4. Instrument nach Anspruch 3,
**dadurch gekennzeichnet, dass** die Hakenmodule (20) durch die Stellmittel (26, 28, 30) freigebbar sind, so dass sie durch die elastische Rückstellkraft der Haut in die in Richtung der Zugkraft zurückgezogene Stellung bewegt werden.

5. Instrument nach Anspruch 3,
**dadurch gekennzeichnet, dass** die Hakenmodule (20) durch die Stellmittel (26, 28, 30) zwangsweise in die in Richtung der Zugkraft zurückgezogene Stellung bewegbar sind.

## Claims

1. An instrument for stretching the tissue of the skin, comprising jaws (10) having hook modules (20) that can be inserted into hook receivers (18) of the jaws (10), and that in each case bear at least one hook (22), wherein the jaws (10) can be moved towards one another in order to introduce a tensile force into the skin via the hooks (22) inserted into the skin, and wherein the hook modules (20) are disposed in the hook receiver (18) in a row transversely to the direction of the tensile force, and the hook modules (20.1, 20.3, 20.5 and 20.2, 20.4) disposed in each case in a hook receiver (18) in the row running transversely to the direction of the tensile force are divided into at least two groups,
**characterised in that** the hook modules (20.1, 20.3, 20.5) of the one group are disposed in the hook receiver (18) staggered in relation to each other against the hook modules (20.2, 20.4) of the at least one other group in the direction of the tensile force and to switch their mutual positions can be moved towards each other in the direction of the tensile force.

2. An instrument according to Claim 1,
**characterised in that** in each case a hook module (20.1, 20.3, 20.5) of the first group and a hook module (20.2, 20.4,) of the second group are disposed in the hook receiver (18) alternately in the row running transversely to the direction of the tensile force.

3. An instrument according to Claim 2,
**characterised in that** the hook modules (20) can be adjusted under compulsion with adjustment means (26, 28, 30) in their position advanced in the direction of the tensile force.

4. An instrument according to Claim 3,
**characterised in that** the hook modules (20) can be released by the adjustment means (26, 28, 30), so that they can be moved by the elastic restoring force of the skin into the position retracted in the direction of the tensile force.

5. An instrument according to Claim 3,
**characterised in that** the hook modules (20) can be moved by the adjustment means (26, 28, 30) under compulsion into the position retracted in the direction of the tensile force.

## Revendications

1. Instrument pour étirer le tissu de la peau, comprenant des mâchoires (10), des modules de crochet (20) pouvant être insérés dans des logements de crochet (18) des mâchoires (10) et portant chacun au moins un crochet (22), les mâchoires (10) pouvant être déplacées les unes par rapport aux autres pour introduire une force de traction dans la peau par l'intermédiaire des crochets (22) piqués dans la peau, et dont les modules de crochet (20.1, 20.3, 20.5 et 20.2, 20.4) disposés dans un logement de crochet (18) en une rangée s'étendant transversalement à la direction de la force de traction sont divisés en au moins deux groupes,
**caractérisé en ce que**
les modules de crochet (20.1, 20.3, 20.5) de l'un des groupes sont disposés dans le logement de crochet (18) de façon décalée par rapport aux modules de crochet (20.2, 20.4) d'au moins un des autres groupes en direction de la force de traction, et peuvent être déplacés les uns par rapport aux autres en direction de la force de traction pour inverser leur position respective.

2. Instrument selon la revendication 1,
**caractérisé en ce que**
respectivement des modules de crochet (20.1, 20.3, 20.5) du premier groupe et des modules de crochet (20.2, 20.4) du deuxième groupe sont disposés dans le logement de crochet (18) en alternance dans la rangée s'étendant transversalement à la direction de la force de traction.

3. Instrument selon la revendication 2,
**caractérisé en ce que**
les modules de crochet (20) peuvent être déplacés par force à l'aide de moyens de réglage (26, 28, 30) dans leur position avancée en direction de la force de traction.

4. Instrument selon la revendication 3,
**caractérisé en ce que**
les modules de crochet (20) peuvent être libérés par les moyens de réglage (26, 28, 30) de manière à être déplacés par la force de rappel élastique de la peau dans la position retirée en direction de la force de traction.

5. Instrument selon la revendication 3,
**caractérisé en ce que**
les modules de crochet (20) peuvent être déplacés par force à l'aide de moyens de réglage (26, 28, 30) dans leur position retirée en direction de la force de traction.
